# EUROPEAN PATENT APPLICATION

(11) **EP 1 627 604 A1**
(43) Date of publication of application: **22.02.2006**
(21) Application number: 05024287.4
(22) Date of filing: 05.03.1999
(51) Int. Cl.: A61B 8/12, A61B 5/00

(54) **Optical-acoustic imaging device**

(30) Priority: 05.03.1998 US 76862 P
(62) Divisional of application: 99950325.3
(71) Applicant: Vardi, Gil M., Chesterfield, MO 63017 (US); Spivak, Victor, 27234 Kiriat Bialik (IL)
(72) Inventor: Vardi, Gil M., Chesterfield, MO 63017 (US); Spivak, Victor, 27234 Kiriat Bialik (IL)
(74) Representative: Granleese, Rhian Jane

(57) **Abstract**

A device for imaging an object, comprising:
an optical fiber (8) provided with a core (1), the core having a Bragg grating, the fiber being capable of reflecting light after illuminating thereof by a laser beam, the reflected light being defined by a wavelength having a period corresponding to that of the Bragg grating; and
an ultrasound transmitter for emanating ultrasonic waves, the ultrasonic waves after being reflected by the object being capable of inducing deformations within the core accompanied by modulation of the reflected light which can be detected and used for the creation of an image.

## Description

### I. Related Applications

This application claims priority to U.S. Provisional Application No. 60/076,862, filed on March 5, 1998.

### II. Field of the Invention

The present invention relates to an omnidirectional imaging device for vascular or nonvascular imaging that may be used as an intravascular guidewire.

### III. Background of the Invention

Intra-vascular and non-vascular imaging are very important techniques that provides information that is not available by angiographic imaging methods such as information about the composition of the subject vessel wall, plaque analysis, and disease processes. It is also very important as an aid to vascular interventions, especially stent deployment.

Prior art intra-vascular ultrasound (IVUS) devices are described as generally adapted to be used via catheter, and are primarily either mechanical or solid state. In the mechanical IVUS catheter, image scanning is accomplished by a rotating drive shaft causing mechanical rotation of a miniature acoustical transmitter. The drive shaft and most of the transmitter are located within the body of a flexible catheter. The design of these devices generally creates difficulties in tracking with a limited image area, and vibration of the catheter during rotation poses a risk to the patient of arterial spasm.

The solid state IVUS catheter does not have a rotating driveshaft, but rather produces images by scanning with electrical impulses that are generated from a large number of piezoelectric elements located within the IVUS. Each piezoelectric element is controlled by a driver such as a computer. Conventional solid state IVUS devices generally have a lumen adapted to receive a guidewire, and a coaxial cable design which enhances the trackability and pushability of the device as compared to the mechanical model.

One deficiency in conventional mechanical and solid state IVUS catheters is the external diameter, generally approximately 1.2 mm. Mechanical limitations on component sizes and noise effects have thus far limited commercially feasible manufacture of a smaller diameter device. In addition, both these devices must be used with traditional intraluminal catherization methods, that is, with the catheter situated over a guidewire.

Some prior art ultrasonic catheter patents describe a thin films of a flexible piezoelectric plastic material, such as poled polyvinyldiene fluoride (PVDF), which can be spot polarized in active regions to serve as piezoelectric transducers. In these devices, the PVDF film is used both as a transmitter and as a receiver. However, it is difficult to adapt this technology to small (less than 1.2 mm diameter) imaging catheters with multiple elements, for several reasons. One such reason is the very low electrical capacitor of each of the receiver elements having a small surface area as compared to the capacitor of the long electrode conductors (more then 1m long). This relationship of elements in the device generally results in a low signal/noise relation. While the signal to noise ration may be increased by the use of preamplifiers near the receivers, physically accommodating the preamplifiers inside of a space with an outer diameter of less than 1.2 mm is very difficult. Another reason is the large signal cross talk experienced due to the long, closely clustered conductors within the device.

Other relevant prior art technology that couples ultrasonic waves with an optical fiber in an intravascular device includes a transducer which is precisely located on thin slab of piezoelectric material. The transducer generates ultrasonic acoustic surface waves that propagate on the surface or within the bulk of the slab. These devices are limited, however, in that they generate doppler signals and not images, and their probing range is limited to the area just in front of the catheter pass. Also, the piezoelectric chip is not small enough to be used in a device with a profile diameter of less than 1 mm, and more importantly, less than .5 mm.

In most commercially available piezoceramic and PVDF IVUS devices, one significant problem is the difficulty in constructing ultrasound imaging catheters with a diameter of less then approx. 1mm., and where the signal to noise ratio will be high enough for the device to be easily used. Such devices are also difficult to manufacture from a mechanical perspective, using conventional components.

Accordingly, it would be useful to have an intra-vascular ultrasound imaging device with a profile of less that approximately 1mm in diameter and most preferably less than .5 mm in diameter, with a signal/noise ratio that is higher than those generated by conventional IVUS devices such as those described above. It would also be useful to have an imaging device for non-vascular applications which demand a device profile of less than 1mm.

### IV. Summary of the Invention

The present invention is a guidewire imaging device for vascular or non-vascular imaging utilizing optico-acoustical methods, which device has a profile of less than 1 mm in diameter, and most preferably less than .5 mm in diameter. The imaging device of the invention comprises a single-mode optical fiber with at least one Bragg grating and a piezoelectric or piezoceramic jacket, which device may achieve omnidirectional (360°) imaging. The imaging guidewire of the invention can function as the guidewire for vascular interventions, and can enables real time imaging during balloon inflation and stent deployment, thus will provide clinical information that is not available when catheter-based imaging systems are used. The device of the invention may enable shortened total procedure times, including the fluoroscopy time, and will also reduce radiation exposure to the patient and the operator.

Thus, it is an object of the invention to provide an optico-acoustic device for vascular or non-vascular imaging with a profile of less than 1 mm, and most preferably less than .5 mm.

Another object of the invention is to provide a guidewire imaging device for producing real time images during vascular intervention procedures prior to catheter insertion and throughout the procedure.

A further object of the invention is to provide a device which is capable of omnidirectional 360 degree imaging.

Still another object of the invention is to provide an intravascular imaging technique with an improved signal to noise ratio over prior art intravascular imaging devices.

### V. Description of the Figures

Fig. 1 is a schematic diagram of a conventional optical fiber.
Fig. 2 is schematic diagram of a PVDF jacketed optical fiber.
Fig. 3 is a schematic diagram of a Fiber Bragg Grating based sensor.
Fig. 4 is a schematic depiction of a PVDF and FBG based ultrasound pulser-receiver of the invention.
Fig. 5 is a schematic diagram of a PVDF and FBG based ultrasound pulser-receiver having a plurality of Bragg gratings.
Fig. 6 is a schematic diagram of a PVDF and FBG based ultrasound pulser-receiver having a plurality of variable Bragg gratings.
Fig. 7 is a schematic depiction of the optical fiber of the invention with a strength member.
Fig. 8 is a schematic depiction of an ultrasound imaging catheter of the invention.
Fig. 9 is a schematic depiction of a catheter of the invention provided with a spiral strength member.
Fig. 10 is a schematic depiction of a fiber with bead-shaped cladding.
Fig. 11 is a schematic depiction of a fiber of the invention with bead cladding and with a spiral strength member.
Fig. 12 is a device of the invention with bead cladding and with ribs.
Fig. 13 is a catheter of the invention adapted with lens apertures.

### VI. Description of the Preferred Embodiments

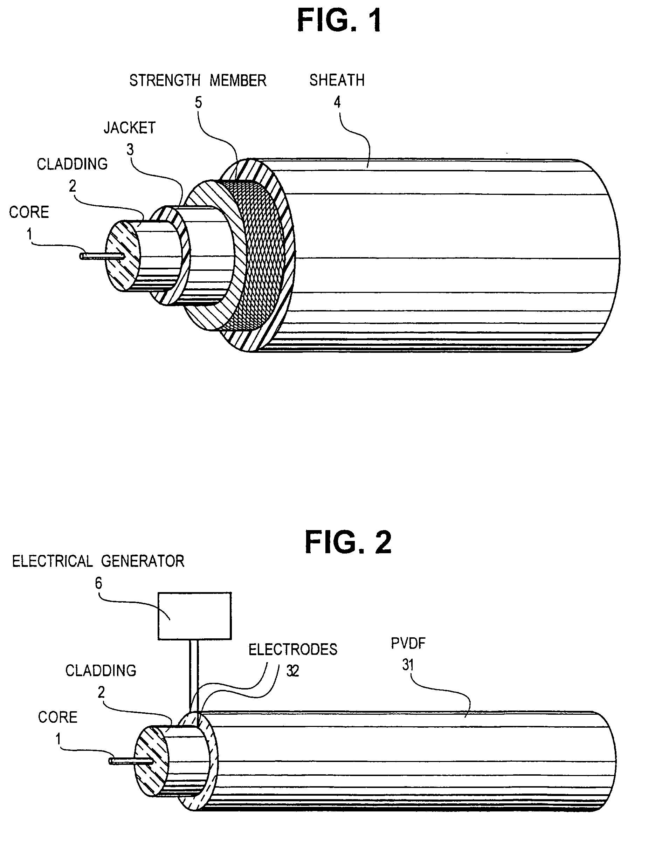

The device of the invention utilizes a single optical fiber, for example but not limited to a glass fiber at least partly composed of silicon dioxide. The basic structure of a generic optical fiber is illustrated in fig. 2, which fiber generally consists of layered glass cylinders. There is a central cylinder called the core 1. Surrounding this is a cylindrical shell of glass, possibly multilayered, called the cladding 2. This cylinder is surrounded by some form of protective jacket 3, usually of plastic (such as acrylate). For protection from the environment and more mechanical strength than jackets alone provide, fibers are commonly incorporated into cables. Typical cables have a polyethylene sheath 4 that encases the fibers within a strength member 5 such as steel or Kevlar strands.

Optical fibers can be broadly classified according to their refractive index profile and dimensions. The invention described below uses single-mode fibers.

Fig. 2 shows an optical fiber coated by a piezoelectric jacket, to which an alternating voltage electrical generator 6 is attached to electrodes 32 situated on either side of the jacket, the generator 6 sends electrical impulses to the electrodes 32, which impulses cause mechanical oscillations in the jacket 31.

In recent years Fiber Bragg Grating (FBG) sensors have generated great interest because of their potential use in a wide range of applications such as telecommunications. FBGs form an integral part of the optical fiber structure and can be written intracore during manufacture or after manufacture.

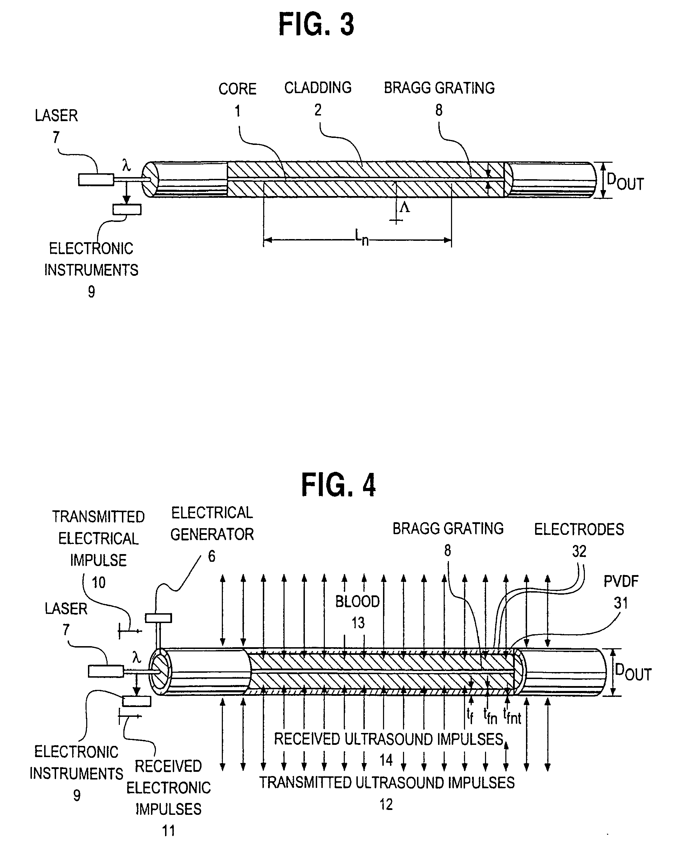

As illustrated in Fig. 3, when illuminated by a broadband light laser 7, a uniform pitch Fiber Bragg Grating ("FBG") element 8 will reflect back a narrowband component centered about the Bragg wavelength λ given by λ=2*n*Λ, where n is the index of the core of the fiber and Λ represents the grating period. Using a tunable laser 7 and different grating periods (each period is approximately .5µ) situated in different positions on the fiber, it is possible to make independent measurement in each of the grating positions.

### Example 1:

One preferred embodiment of the invention is illustrated in FIG. 4. This embodiment includes a single-mode optical fiber with a Bragg grating 8 and a piezoelectric or piezoceramic jacket 31. The jacket may be any suitable piezoelectric or piezoceramic material, and one preferable material is poled PVDF. It is contemplated that other jacket materials will work with the invention, so long as the material has suitable flexibility and piezoelectric characteristics.

In the preferred embodiment of the device of the invention as illustrated in Fig. 4, an electrical generator 6 transmits ultrasound impulses 10 to both the Bragg grating 8 and to the outer medium 13 in which the device is located, for example, the blood. Primary and reflected impulses 11 are received by the Bragg grating 8 and recorded by electronic instruments 9 using conventional methods, such as by a photodetector and an oscilloscope. From the recorded signals, a corresponding image is generated by conventional methods. Hence, the invention utilizes omnidirectional sonar (pulser-receiver) at each of the imaging locations. If mechanical deformations appear inside the optical fiber, they cause modulation of light reflected backward, which is received by the electronic instruments 9.

It is contemplated that in the various devices constructed according to the invention, the thickness of the jacket as well as the diameter of the optical fiber may vary significantly, and the only requirement is that the entire device be less than 1mm and most preferably less than 300µ, and that the signals generated by the device are suitable to generate an image.

The ultrasound transmitter device of the invention comprises a single fiber covered by a piezoelectric active (poled) PVDF jacket has a total outside diameter of preferably less than 1mm, and most preferably less than 300µ. It is also contemplated that devices may be made in accordance with the principles of the invention with profiles of approximately or less than 200µ. Devices with other frequency transmitters may also be constructed in accordance with the principles of the invention, as applications dictate. The device of the invention includes any needed frequency of transmitter.

### Example 2

It may also be possible to expand the frequency band of the signal by using a damped silica fiber. In this variation of the preferred embodiment of the invention, frequency band expansion causes shortening of the signal in time, which improves the resolution of the received signal. For instance, using a damped fiber in a device of the invention, we have obtained maximum widths of the frequency band of the signal of approximately 110, although another variations will be achieved depending upon experimental conditions. If damped fibers are utilized, transmitters transmitting at less than 40 MHz may be used.

### Example 3

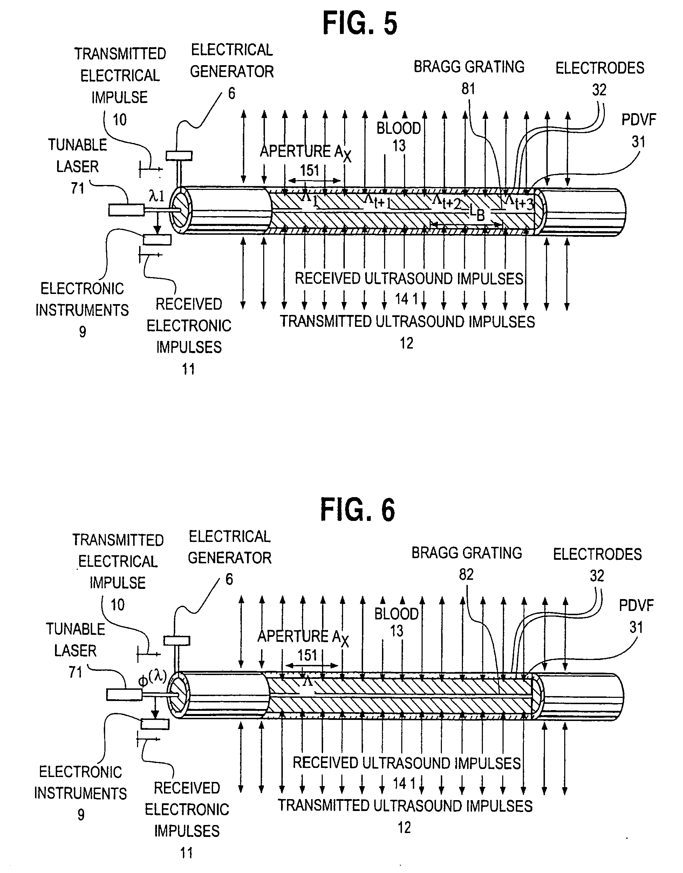

As shown in Fig. 5, one other preferred embodiment of an imaging device in accordance with the invention comprises a plurality of Bragg gratings 81 with different periods, each period being approximately .5µ. By using multiple Bragg gratings, a set of distributed sonars are obtained. By utilizing a tunable laser 71 as previously described, we obtain scanning over an omnidirectional array. A Bragg grating length L_{B} of some hundreds of optical wavelengths are sufficient to reflect considerable part of the optical beam. The ultrasound impulses 141 are received only by the Bragg gratings 81, with the period of Λᵢ which is equal to the aperture Aₓ.

### Example 4

In yet another preferred embodiment of a device of the invention as illustrated in Fig. 6, instead of a plurality of Bragg gratings, the device may incorporate a single variable grating, with a variable period. When a tunable laser is adjusted to the wavelength λ₁ the receiving element is the Bragg grating. When the laser wavelength is adjusted to other wavelengths λ_{2- 6...}, the corresponding positioning of the Bragg grating along the axis of the fiber is also adjusted.

We have determined that for a device with a 40 MHz frequency transmitter and aperture Aₓ =151-200µ, the reception obtained by the invention provides acceptable imaging.

### Example 5

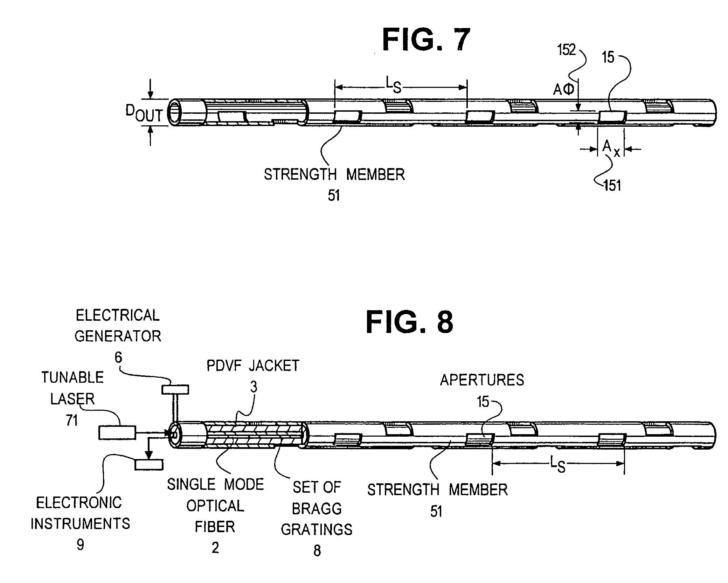

In yet another preferred embodiment of the device of the invention as illustrated in Fig. 7, a strength member may be optionally added. This strength member is very thin, and even with the strength member, it is contemplated that the device of the invention is still less than 1 mm in diameter.

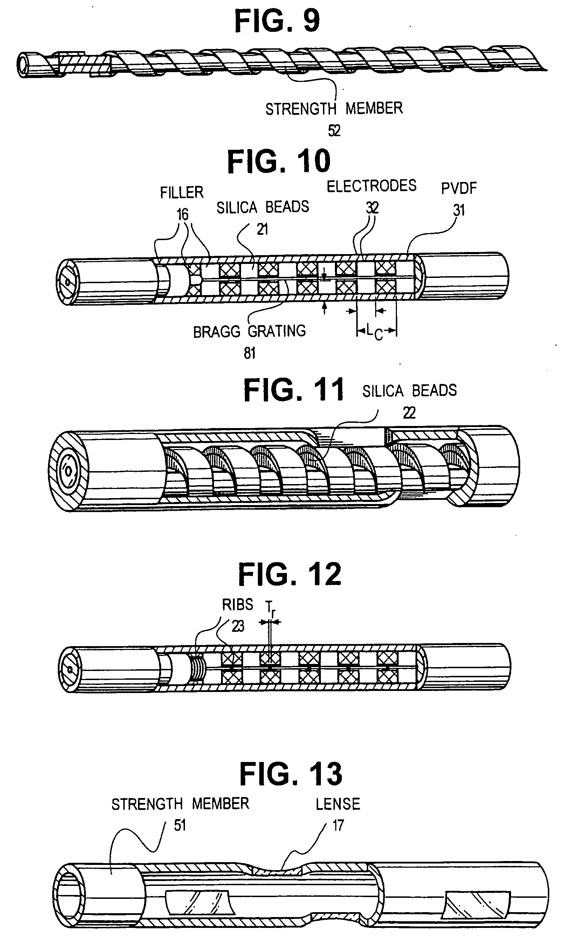

To preserve the omnidirectional scanning ability of the invention, the optical fiber is placed into the strength member 51 comprising a plurality of rectangular apertures 15. These apertures 15 have a length dimension 151 along axis x = Aₓ, and a circumferential dimension length 152 = A_{ϕö}152. In a preferred embodiment the apertures are rectangular, although other shapes may be utilized. The apertures 15 may be distributed throughout the imaging portion of the device, and may be distributed in a pattern, for example a spiral as illustrated in Fig. 9.

### Example 6

Example 6, as illustrated in Fig. 8, is a catheter version of the device of the invention, which produces ultrasound scanning both along the axis and along the circumference. It is comprised of a single mode optical fiber 2 with a plurality of Bragg gratings 8. The optical fiber is provided with a jacket 3, and a strength member 51, which has set of apertures 15. The strength member may be made of any hard, flexible and durable biocompatible material such as metal. Apertures are placed uniformly on the surface of strength member, both along the length and angle. The outside diameter of this device is less than 1mm, and most preferably less than .5 mm. It is contemplated that the device may further have a most preferred outer diameter of less than 400µ. The apertures may be constructed using conventional photochemistry technology.

As illustrated in figure 8, the device is shown with an array of apertures Aₓ=A_{ϕö}=200µ, period Lₛ=1000µ. By applying electrical impulses to the electrodes of PVDF jacket 3 from electrical generator 6 we generate acoustical impulses in the all apertures simultaneously. The ultrasound impulses will expand in a direction perpendicular to the optical fiber surface, and reflect back from the nonhomogeneous medium (tissue). By tuning the laser 71, it is possible to realize scanning of the received ultrasound signals. Electronic instruments 9 receive, process and displaying the resulting images. One can estimate the scanning period L_{S} of scanning as 0.5 to 1.0 mm lengthwise and number of directions around the fiber as 5 to 10.

### Example 7

The design of the invention may also comprise more than one optical fiber. If there are a plurality of fibers within the strength member, it is possible to decrease the period and increase the number of directions of the scanning.

### Example 8

Fig. 9 shows a variation of the strength member 52, comprising a spiral strength member. Use of this member is believed to produce smoother scanning, and a simpler manufacture than a strength member with apertures.

### Example 9

As illustrated in Fig. 10, another variation of the device of the invention is a variable diameter cladding, preferably of silica, with a period L along the fiber. This variation is achieved by the use of beads 21, which causes an increased sensitivity to acoustical waves. Maximum efficiency is achieved if the period L_{c} is equal to one of the following resonance lengths: it is approximately equal to acoustical wavelength in water L_{c1} Å (1500/40·10⁶)=37.5·10⁻⁶m (for 40 MHz); or it is equal to the quasi-Lamb wavelength in the silica fiber L_{c2}.

In this embodiment, the Bragg grating interacts with optical waves and with the acoustical grating formed by the beads.

### Example 10

As illustrated in Fig. 10, an additional increase in sensitivity f the device may optionally be received if a filler 16 is used to fill the gaps between the beads.. This filler is produced from material with comparatively low acoustical impedance, such as a solid polymer, gel, fluid or other suitable material. For the purpose of yet additional increasing in sensitivity, gap filling filler is selected from the materials which sound velocity c_{f} lower than sound velocity in water (blood), that is c_{f}<1500m/sec. One example of such materials is silicon rubber having the sound velocity c_{f}~1000m/sec. In consequence of the sound velocity difference the energy focusing is achieving. Thus, the filling material functions as a signal collecting lens.

### Example 11

Yet another variation of the device of the invention includes a spiral jacket 22, as shown in Fig. 11.

### Example 12

Another embodiment (illustrated in Fig. 12) includes adding ribs 23 to the jacket In one example of a device with ribs, to achieve 40 MHz resonance, silica ribs should nave approximately dimensions: height *H*_{*r*}=10 microns and thickness *T*_{*r*}=*4.5* microns. The oscillations of ribs 23 induce the additional deformations at the fiber axes, hence causing the increasing in sensitivity. It is possible to fabricate ribs by conventionally known micromachining technology.

In a deviation of the ribbed embodiment, the ribs may have varying thicknesses, which are believed to lead to acoustical damping, and hence an increase in bandwith and resolution. If each of the ribs 23 will have different height *H*_{*r*}. and width *T*_{*r*} then they will resonate at different frequencies.

### Example 13

For the purpose of yet additional increases in sensitivity, the apertures of the strength member may filled with a material with a velocity c_{L}> 1500m/sec, and an outside surface curvature which forms a focused lens, as illustrated in Fig. 13.

It is thus seen from the above description of the preferred embodiments that the objects of the invention are attained. Variations on this embodiment will be apparent to those skilled in the art without departing from the scope of the invention. All matter contained in the above description and the accompanying drawings is intended to be illustrative of the invention, and not limiting in the scope of the invention.

## Claims

1. A device for imaging an object, comprising:
an optical fiber (8) provided with a core (1), the core having a Bragg grating, the fiber being capable of reflecting light after illuminating thereof by a laser beam, the reflected light being defined by a wavelength having a period corresponding to that of the Bragg grating; and
an ultrasound transmitter for emanating ultrasonic waves, the ultrasonic waves after being reflected by the object being capable of inducing deformations within the core accompanied by modulation of the reflected light which can be detected and used for the creation of an image.

2. The device of claim 1, in which the optical fiber is embodied in an elongate member that includes sufficiently small diameter and sufficient rigidity to permit use for introducing an ancillary device into the body.

3. The device of claim 1, in which the optical fiber is embodied in an elongate imaging guidewire that includes sufficiently small diameter and sufficient rigidity to permit use for introducing an ancillary device into vasculature of the body over the imaging guidewire.

4. The device of claim 2, in which the optical fiber is embodied in an elongate imaging guidewire having a diameter of less than about 1 millimeter.

5. The device of claim 4, in which the optical fiber is embodied in an elongate imaging guidewire having a diameter of less than about 0.5 millimeters.

6. The device of claim 5, in which the optical fiber is embodied in an elongate imaging guidewire having a diameter of less than about 0.2 millimeters.

7. The device of claim 1, in which the optical fiber is embodied in an elongate member that includes a plurality of Bragg grating at different locations along a length or circumference of the elongate member to provide three dimensional imaging about a circumference of the elongate member.

8. The device of claim 1, comprising at least one aperture located in a relationship with respect to the Bragg grating so as to pass through the aperture ultrasonic waves reflected by the object.

9. The device of claim 8, in which the aperture has a length, in a longitudinal direction of the optical fiber, of approximately between about 151 micrometers and about 200 micrometers.

10. The device of claim 8, including a plurality of apertures disposed in a spiral pattern along an elongate member in which the optical fiber is embodied.

11. The device of claim 1, in which the Bragg grating includes a length, in a longitudinal direction of the optical fiber, which is at least 200 times an optical wavelength of light passing through the core of the optical fiber.

12. The device of claim 1, in which the Bragg grating includes a variable period.

13. The device of claim 12, further including a tunable laser, optically coupled to the variable period Bragg grating, the laser operable to adjust an effective location of sensing by the Bragg grating by adjusting a wavelength of light to select a desired portion of the Bragg grating that provides a corresponding desired period.

14. The device of claim 1, in which a diameter of a cladding about the core of the optical fiber varies longitudinally.

15. The device of claim 14, in which the diameter of the cladding varies periodically with a period that is approximately equal to an acoustic wavelength in water.

16. The device of claim 14, in which the diameter of the cladding varies periodically with a period that is approximately equal to a quasi-Lamb wavelength in the optical fiber.

17. The device of claim 1, comprising a display for forming an image of a region within the object using the modulated reflected light.

18. The device of claim 1, further comprising an electrical-to-acoustic transducer, at least a portion of the electrical-to-acoustic transducer located away from a proximal end of the optical fiber.

19. The device of claim 18, in which the electrical-to-acoustic transducer includes a piezoelectric jacket about at least a portion of the optical fiber.

20. The device of claim 19, in which the piezoelectric jacket is substantially formed from polyvinyldiene fluoride (PVDF).

21. The device of claim 19, in which the piezoelectric jacket is shaped as a spiral about the optical fiber in a longitudinal direction of the optical fiber.

22. The device of claim 19, in which the piezoelectric jacket includes a plurality of ribs.

23. The device of claim 1, comprising an acoustic signal collecting lens.

24. The device of claim 1, further comprising a tubular strengthening member, the member being provided with at least one aperture located in a relationship with respect to the Bragg grating so as to pass the ultrasonic waves which have been reflected by the object therethrough.

25. A device for imaging comprising:
a guidewire;
an optical fibre;
an ultrasound transmitter; and
at least one Bragg grating sensor;
wherein the optical fibre is located around the guidewire and where at least one Bragg grating is contained within the optical fiber, and wherein the profile of the device is less than 100 mm in diameter.
